Europäisches Patentamt

European Patent Office

Office européen des brevets

(11) Publication number: **0 246 643**
**A2**

# EUROPEAN PATENT APPLICATION

(21) Application number: **87107365.6**

(51) Int. Cl.⁴: **G01N 33/543**

(22) Date of filing: **20.05.87**

(30) Priority: **20.05.86 US 865378**

(43) Date of publication of application:
**25.11.87 Bulletin 87/48**

(84) Designated Contracting States:
**AT BE CH DE ES FR GB GR IT LI LU NL SE**

(71) Applicant: DNA PLANT TECHNOLOGY
OPERATING CO. (under the laws of the State
of Delaware)
2611 Branch Pike
Cinnaminson, New Jersey(US)

Applicant: KOPPERS AGRI-RESEARCH
COMPANY (under the laws of the State of
Delaware)
2611 Branch Pike
Cinnaminson, New Jersey(US)

(72) Inventor: Grothaus, G. David
C-22 Tenbytowne Apts.
Delran New Jersey(US)
Inventor: Stocker, Dennis R.
11 Ardsley Road
Yardley Pennsylvania(US)

(74) Representative: Schmidt-Evers, Jürgen,
Dipl.-Ing. et al
Patentanwälte Dipl.-Ing. H. Mitscherlich
Dipl.-Ing. K. Gunschmann Dipl.-Ing.
Dr.rer.nat. W. Körber Dipl.-Ing. J.
Schmidt-Evers Dipl.-Ing. W. Melzer
Steinsdorfstrasse 10
D-8000 München 22(DE)

(54) Method of preparing diagnostic test dipsticks.

(57) Antibodies are covalently bonded to a porous activated membrane (6), particularly a porous activated nylon membrane, and the membrane (6) is affixed to the end of an elongated substrate (4) to provide a dipstick (2) which may be usefully employed in performing a diagnostic test for diseases, especially plant diseases.

## FIG. IB

## METHOD OF PREPARING DIAGNOSTIC TEST DIPSTICKS

This invention generally relates to the diagnosis of plant diseases; and, more specifically, to a method especially well suited for the field diagnosis of plant diseases, and to a kit including the materials needed to carry out that method and more particularly, to the solid support members, often referred to in the art as "dipsticks", employed in the diagnostic test method and their method of preparation.

Plant diseases cause a tremendous amount of damage and economic loss each year. For example, if certain types of grass diseases, such as pythium blight, become wide spread over a particular area, it may be necessary to remove and to replace all the grass in the affected area. Even if this is not necessary, an extensive amount of time and treatment may be required to control the disease.

The magnitude of the problem is caused, in part, by the fact that heretofore it has been very difficult or prohibitively expensive to diagnose most plant diseases until the symptoms of the diseases became visible. In particular, very few plant diseases can be diagnosed in a laboratory before the symptoms of the diseases become visible in the field, most such laboratory tests are expensive and time consuming. Consequently, these tests are not normally performed until there is visible evidence in the field that a plant may be infected; and as a result, in most instances, by the time a disease is finally diagnosed, it is quite widespread.

Numerous comparatively simple and inexpensive diagnostic tests have been successfully developed using antibodies to detect human and animal diseases in very early stages of infection. To do this, a first antibody, which is reactive with a selected antigen that is, or is associated with, a disease causing organism, may be immobilized on a solid support member; and then a sample liquid solution, which is suspected of harboring the selected antigen, is contacted with the immobilized antibody. If the selected antigen is present in the sample liquid solution, the antigen reacts with and becomes bound to the first antibody to form an antigen-antibody binary complex on the solid support member.

After washing the unreacted material from the support member, the binary complex is contacted with a second, labeled antibody that is also reactive with the selected antigen. If the binary complex is present on the solid support, this second antibody reacts with and becomes bound to the antigen component of the complex to form an antibody-antigen-antibody tertiary complex. After washing the solid support member to remove any of the second antibody that did not react with the selected antigen, the support member is tested for the second antibody by any of a variety of analytical techniques.

Diagnostic procedures of the above-outlined type have been successfully developed using polyclonal or monoclonal antibodies to detect antigens associated with particular diseases with a very high degree of reliability. Monoclonal antibodies are made via a process, referred to as hybridoma technology, in which hybridoma are formed by the fusion of short-lived antibody producing cells (usually spleen cells) and long-lived myeloma cells to produce long-lived antibody synthesizing cell lines. Each hybrid cell line produces a unique and characteristic antibody that has the ability to bind, with a very high degree of specificity, to a single type of antigen.

The cells that are fused to form a particular hybridoma cell line can be selected or treated so that the monoclonal antibody synthesized by that cell line will bind only to a chosen antigen. If such an antibody is used in the above-discussed procedure as either the first or second antibody, then the antibody-antigen-antibody tertiary complex will form on the solid support member, with a very high degree of accuracy, if and only if the chosen antigen is present in the sample liquid solution. Polyclonal antibodies with the appropriate affinity and specificity may also be used to detect antigens with a very high degree of accuracy.

Procedures of the above-outlined general type have been successfully employed on a commercial basis in the laboratory to test for human and animal diseases, among other things. Because of the relative simplicity and accuracy of procedures of this type, it would be very desirable to provide similar tests to diagnose plant diseases. Unique problems have been encountered, though, in developing commercially practical procedures, of the above-discussed general type, for the field diagnosis of plant diseases. For instance, it has been difficult to develop a simple and inexpensive yet field-effective technique for preparing a suitable liquid solution containing an effective quantity of a plant material that may be tested to indicate reliably whether the plant is infected with a particular antigen. A commonly assigned patent application, filed concurrently herewith is directed to providing a plant disease diagnostic procedure that is very well suited for use in the field.

U.S. Patent 4,135,884 of Shen discloses a test stick for use in a clinical assay wherein an elongated member of molded plastic such as polypropylene is coated at its end with an antibody. The test portion of the stick is then immersed into a test tube containing a solution of an antigen to effect antibody-antigen reaction. Upon removal from the test tube the test stick is evaluated to determine the extent of said reaction, for example, by means of radioactivity where the antigen is labelled with a radioactive tracer.

U.S. Patent 4,168,146 of Grubb discloses a test strip for carrying out immunoassays. The test strip is composed of a bibulous carrier to which antibodies are bound. The test strip is wetted with an aqueous solution containing a suspected antigen. Following this the test strip is contacted with an aqueous solution containing labelled antibodies. The bibulous carrier is composed of porous capillarity-possessing materials, for example, filter paper, chromatographic paper and cellulose acetate film or discs, as well as a three dimensional network of dextran chains cross-linked with epichlorohydrin and plastic materials such as polyvinylchloride and combinations such as polyvinylchloride-silica. The antibodies are bound to the bibulous carrier by adsorption, absorption or covalent binding by use of a cyanogen halide or glutaraldehyde.

U.S. Patent 4,444,880 of Tom discloses a dipstick immunoassay employing a bibulous solid support where production of a dye is the means for detecting the presence of an analyte. The support is impregnated with metaperiodate and a dye precursor to provide a detectable signal. The bibulous support may be a cellulosic support such as paper. In a preferred embodiment, the paper is activated to provide stable covalent bonding between the paper and the material, such as antibodies, being bound to the support.

This invention relates to a process of preparing a dipstick having antibodies immobilized thereon which comprises:

(a) contacting a porous membrane, effective to covalently bond with antibodies, with a solution of antibodies under conditions effective to covalently bond such antibodies to said porous membrane,

(b) drying said porous membrane and,

(c) affixing.said porous membrane on a longitudinal end of an elongated substrate.

In a preferred embodiment of this invention, the membrane having the antibodies bound thereto is contacted with a blocking solution to inactivate those sites not occupied by the antibodies before the membrane is affixed to the end of the elongated substrate.

This invention is also directed to dipsticks prepared by the above described processes.

Figures 1A and 1B are schematic views of the dipstick of the present invention.

Figure 2 is a schematic view showing equipment used to carry out the diagnostic method employing the dipstick of the present invention.

Figure 3 is a perspective view of a kit, including the dipstick of this invention, that may be used to practice the diagnostic test described herein.

The subject invention relates to the preparation of dipsticks having antibodies immobilized on the end thereof which may be usefully employed in performing diagnostic tests to determine the presence, or absence, particularly, of plant diseases. Briefly, the antibody is bonded to a porous membrane which is then attached to the end of an extended substrate known in the art as a dipstick.

The membrane employed in the present invention must be a porous material which will form a covalent bond with an antibody. A particularly preferred porous membrane is an activated nylon membrane commercially available from Pall Corporation, Glen Cove, New York. One such product is described as Pall Biodyne Affinity Membrane having a 0.65 um pore size. Although there are a number of porous materials to which antibodies may be attached, in most instances the manner of attachment may be described as absorption, adsorption or hydrostatic in nature, all of which do not provide a bond as strong as a covalent bond. Thus, the antibodies held to the porous material by these other bonding mechanisms are more subject to removal from the porous material by outside forces such as the soaking or washing with aqueous solution which is employed during the performance of a diagnostic test. It is preferred, therefore, that a covalent bond be formed when preparing dipsticks in accordance with the present invention.

In commercialization of plant diagnostic test kits, it is essential that the materials necessary to conduct the required test procedures are capable of being mass produced. Where dipsticks are employed in a diagnostic test they must be so designed that they can be produced in large quantities at a cost-effective price. The dipstick of the present invention, which employs a small section of a porous membrane as the antibody support which in turn is affixed to an inexpensive elongated substrate, is readily adapted to cost-effective mass production procedures.

Antibodies are immobilized on the porous membrane preferably in a simple procedure which assures the preparation of a substantially clean and uncontaminated product. Workers should wear gloves during the entire procedure of dipstick preparation. A vacuum impregnation procedure is ideally suited to this preparation which essentially involves the simultaneous preparation of a number of antibody-impregnated membranes from a sheet of activated nylon membrane. Essentially, the sheet is placed on a template containing a number of wells and an aqueous solution is applied to the membrane in the vicinity of each of the wells and permitted to soak through the membrane into the well. Then the membrane is subject to a vacuum drying which promotes the covalent bonding of the antibodies to the porous membrane. Following this, the impregnated membrane is soaked in a blocking solution to inactivate those sites in the membrane not occupied by antibodies so that these unoccupied sites are not available to non-specifically bind other materials with which the membrane will come into contact during the diagnostic test. The blocking solution employed may be any of those known in the art. One that is preferred is a 5% solution of non fat dry milk in a tris buffered saline solution, preferably including an effective quantity of antifoam agent. (The tris buffered saline solution is described hereinafter.) After the blocking operation the membrane is subdivided into small segments, each of which contain a section of imbedded antibodies.

The section of porous membrane containing the antibodies is then affixed to the end of an elongated substrate. The elongated substrate may be prepared from a variety of materials, although it is, of course, essential that the material selected must be one which would not adversely affect the diagnostic test. The substrate may be rigid or flexible and may be composed of such materials as wood, plastic and the like. Preferred materials are non-wetting plastics such as polypropylene, poly (ethylene terphthalate) and poly (vinyl chloride). Particularly preferred is poly (ethylene terphthalate) available commercially in the form of Mylar sheets. The elongated substrate may be about 0.75 to about 1.25 cm, preferably about 1 cm in width and about 10 to about 15 cm, preferably about 13 cm in length prepared from a thin flexible sheet of Mylar plastic.

The prepared membrane is affixed near one end of the dipstick by means of adhesive. The adhesive must also be of a material which will not adversely affect the diagnostic test. A particularly effective means of affixing the membrane to the elongated substrate is by means of a double sided adhesive tape where one side is affixed to the membrane and the other side to the elongated substrate.

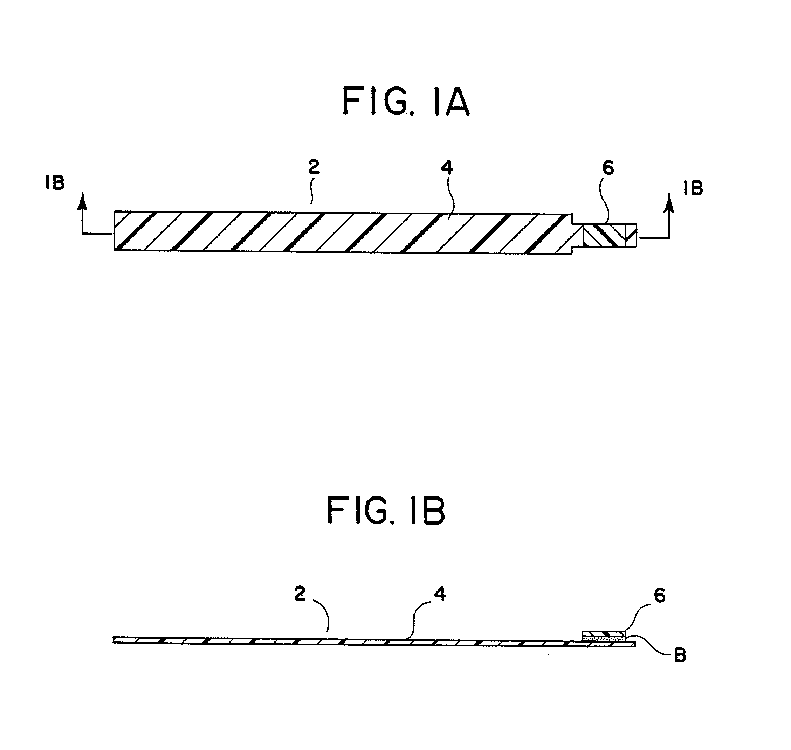

Referring to Figures 1A and 1B, Figure 1A is a plan view of the completed dipstick while Figure 1B is a section view through the longitudinal axis of the dipstick as indicated by the 1B arrows in Figure 1A. The completed dipstick 2 comprises the elongated substrate 4 with porous membrane 6 affixed to the end thereof by means of adhesive 8. Adhesive 8 and membrane 6 are not shown to scale in Figure 1B so as to indicate the construction details more clearly. Membrane 6 is preferably centrally located about the longitudinal axis of substrate 4 to facilitate the evaluation of membrane 6 at the conclusion of the diagnostic test by means of a reflectometer. By being centrally positioned on substrate 4, the membrane will be more properly positioned within the reflectometer so as to permit a more precise evaluation, particularly if a series of dipsticks are to be evaluated and compared.

In a particularly preferred procedure for dipstick preparation in accordance with the invention, a section of activated nylon porous membrane is wetted with deionized water until the entire membrane is wetted. Then the membrane is soaked in tris buffered saline solution (TBS) for about 10 to about 20 minutes. TBS is an aqueous saline solution containing about 500 mM NaCl and 50 mM tris and has a pH of between about 5 to about 9, preferably about 6.5 to about 7.5. The following formulation is typical of TBS:

|  | Per Liter |
| --- | --- |
| NaCl | 29.22 g |
| Trizma Base | 1.18 g |
| Trizma HCl | 6.35 g |
| pH to 7.5 using Trisma Base or Trisma HCl | |

The wetted membrane is then placed on top of a piece of blotting paper and inserted into a clean vacuum filtration apparatus, such as a Schliecher and Schuell Manifold vacuum filtration apparatus having a template with a multiplicity of wells which serve as the areas for deposition of the antibodies. Vacuum is applied until the TBS wetting the membrane has filtered through the porous membrane. The vacuum is terminated and a quantity of a solution of antibodies is added to each of the wells in the template. The antibody solution may be prepared by diluting partially purified rabbit immunoglobulin in TBS in a dilution of about 1:1000. A typical antibody may be an ammonium sulfate precipated polyclonal rabbit serum. A monoclonal antibody may be employed as well. The antibody solution is allowed to filter through the porous membrane until the template wells are empty. Vacuum is then applied for about 10 to about 20 minutes to dry the membrane. With the vacuum still on, the treated membrane is removed from the vacuum filtration apparatus by means of forceps and allowed to hang dry for about an hour.

The membrane is then re-wetted with deionized water and transferred to a quantity of blocking solution maintained at a temperature of about 35 to about 45°C and allowed to incubate for about 25 to about 35 minutes during which the solution is mildly agitated. A useful blocking solution may be prepared by adding about 50 g of non-fat dry milk and about 0.1 ml of an antifoam agent, such as Dow Antifoam A, to a liter of TBS. After the initial incubation period, the blocking solution is changed and the incubation procedure is repeated. This procedure is then repeated, i.e., four charges of blocking solution, total time about 100 to about 140 minutes.

After the blocking step, the membrane is removed by means of forceps and hang dried for about 15 to about 17 hours at room temperature. Following the drying, the prepared porous membrane may be stored in a closed container with dessicant until applied to the dipsticks.

The dipsticks are prepared from sheets of plastic film cut into the desired shape. Typically, thin Mylar sheets are employed cut into individual strips of about 1.0 cm x 13 cm. To facilitate positioning the dipstick into a reflectometer, one end of the strip may be cut by removing a 0.2 cm x 1.5 cm section from each side of the strip to provide at this end a centrally located section of about 0.6 cm x 1.5 cm to which a treated section of porous membrane will be affixed.

The dipstick blanks are rinsed in deionized water and dried with adsorbent paper towels. A small section of double stick tape, such as 3M brand carpet tape, is applied to the narrow end of each dipstick. The prepared membrane, handled with gloves and forceps, is cut into individual pieces so that each section contains a "dot" of antibody. A piece of porous membrane, containing the antibody, is carefully centered above the piece of adhesive tape of the dipstick blank and firmly pressed onto the tape. The dipsticks are then stored in racks until they are inserted in protective envelopes for assembly into the diagnostic test kits.

The dipsticks of the invention may be usefully employed in a diagnostic method for testing a plant for the presence of a suspected antigen, comprising the steps of taking a sample of a plant, rubbing the sample between first and second extraction pads to extract sap from the sample, and collecting the sap on the first extraction pad. The sap is transferred to a liquid solution from the first extraction pad, and then that liquid solution is tested for the presence of the suspected antigen.

With one embodiment, this diagnostic testing is performed by contacting the liquid solution with a tag antibody reactive with the suspected antigen and with a carrier body, i.e., a dipstick, having attached thereto a capture antibody also reactive with the suspected antigen. (The terms "carrier body", "dipstick" and "solid support member" are used interchangeably herein in referring to and describing the elongated substrate which serves as the support for an antibody.) A tag antibody-antigen-capture antibody complex forms on the carrier body if the suspected antigen is present in the liquid solution, and then the carrier body is tested for the presence of the tag antibody.

The tag and capture antibodies may both be monoclonal antibodies, they may both be polyclonal antibodies, or one may be a monoclonal antibody while the other one is a polyclonal antibody.

A kit for carrying out this plant disease diagnostic test comprises the first and second extraction pads, and a container holding the above-mentioned liquid solution and which is adapted to receive the first extraction pad to transfer sap therefrom to the liquid solution. The kit will normally include the carrier body, a supply of tag antibody, and means to test the carrier body for the presence of the tag antibody. Preferably, all the parts of the kit are neatly and securely packed in a small, easily carried box.

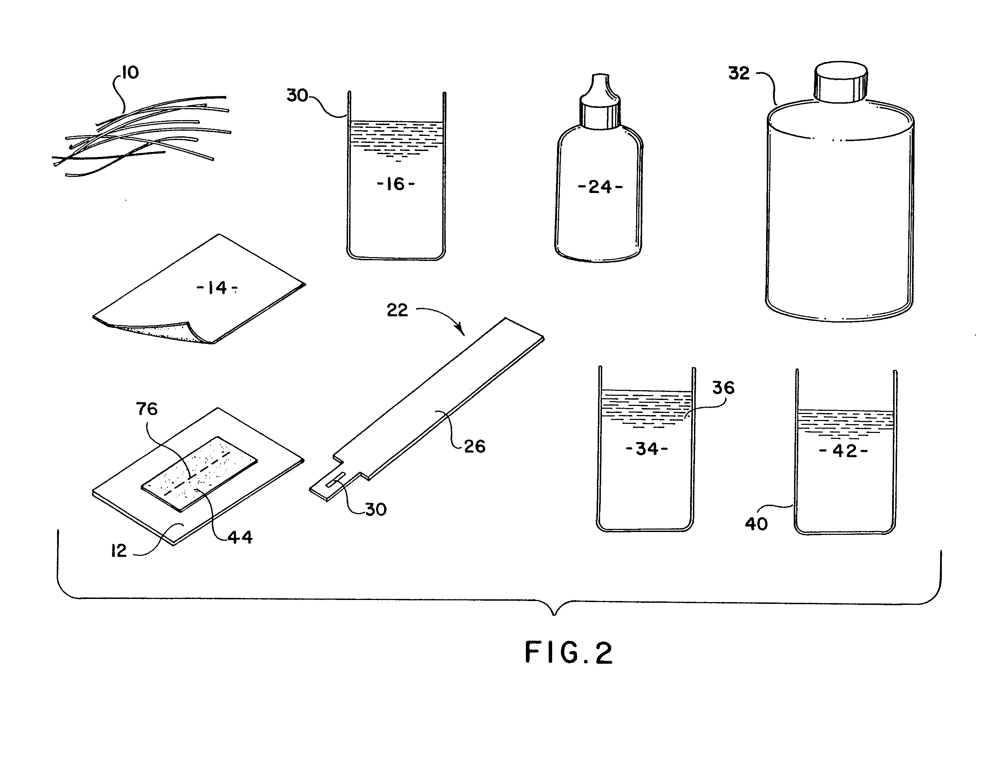

In accordance with the present diagnostic test, and with reference to Figure 2, to test a plant for a suspected antigen, a sample 10 of the plant is taken and rubbed between first and second extraction pads 12 and 14 to extract sap from the sample. The sap is collected on first extraction pad 12 and transferred therefrom to liquid solution 16, which preferably is held within a container or tube 20, and then that liquid solution is tested in any suitable way for the presence of the suspected antigen.

With one embodiment of the diagnostic test when utilizing the dipstick of the invention, solution 16 is tested for the suspected antigen by contacting the liquid solution with a first, labelled antibody reactive with the suspected antigen, and with carrier body 22 having attached thereto a second antibody also reactive with the suspected antigen. The first antibody, which is preferably taken from a solution in bottle 24, is referred to as a tag antibody, and the second antibody is referred to as a capture antibody. If the suspected antigen is present in liquid solution 16, the antigen reacts with and becomes attached to both the tag antibody and the capture antibody to form a tag antibody-antigen-capture antibody complex on or attached to carrier body 22. Carrier body 22 is then tested for the presence of the tag antibody, and any of a variety of analytical techniques may be employed to do this.

Preferably, carrier body 22 used in the practice of the test procedure includes a thin, elongated stick 26, referred to as a dipstick, and a first end of this stick is provided with a membrane 30 that contains the capture antibody. Liquid solution 16 is contacted with this carrier body 22 by simply lowering this first end of the stick into container 20 to immerse membrane 30 into the liquid solution. To insure adequate exposure of the capture antibody to liquid solution 16, preferably membrane 30 is left to soak in the liquid solution for an extended period of time, for example, about two hours.

When carrier body 22 is immersed in liquid solution 16, some tag antibody will normally become attached to the carrier body by means other than a reaction with and attachment to the suspected antigen. Thus, when carrier body 22 is removed from liquid solution 16, normally tag antibody will be on the carrier body even if the suspected antigen is not in the liquid solution and has not become attached to the carrier body. The presence of such unreacted tag antibody on carrier body 22, in the absence of the suspected antigen, is undesirable since it may lead to a false conclusion about the presence of the suspected antigen on the carrier body. Specifically, with the preferred embodiment of the invention described herein, carrier body 22 is directly tested for the tag antibody, with the presence of that antibody indicating indirectly that the suspected antigen is also on the carrier body. Hence, if tag antibody, but not the suspected antigen, is on carrier body 22, a person may mistakenly conclude that the suspected antigen is on the carrier body.

For this reason, preferably carrier body 22 is carefully washed or rinsed after being removed from solution 16 to remove from the carrier body all the unreacted tag antibody --that is, all the tag antibody that is attached to the carrier body by means other than a reaction with the suspected antigen. In this way, all the tag antibody, if any, that remains on carrier body 22 is attached to it via the suspected antigen, and a subsequent test that shows that the tag antibody is on the carrier body reliably indicates that the suspected antigen is also on that carrier body. With a recommended technique, after carrier body 22 is removed from solution 16, the carrier body, specifically membrane 30, is carefully sprayed with a rinse solution from bottle 32, then soaked in solution 34 in container or tube 36 for a period of about 5 minutes, and then again thoroughly sprayed with the rinse solution from bottle 32.

Any suitable procedure may be used to test carrier body 22 for the presence of the tag antibody. For example, the tag antibody may include an enzyme conjugate that reacts with a selected enzyme substrate to produce a product having a particular color, and membrane 30 may be soaked in a container or tube 40 having a solution 42 containing the selected enzyme substrate to see if this color is produced on the membrane 30 as a result of the catalytic activity of the enzyme. If the color is produced, or produced to a certain intensity, the user can conclude that the tag antibody is on carrier body 22, and thus that the suspected antigen is on the carrier body and in plant sample 10 used in the diagnostic test. Conversely, if this particular color is not produced, or not produced to that certain intensity, when membrane 30 is immersed in the solution 42, the tag antibody is not present on carrier body 22, indicating that plant sample 10 is free of the suspected antigen. With this technique, the intensity of the color produced on membrane 30 also is an indication of the degree to which plant sample 10 is harboring the suspected antigen.

If desired, a test may be conducted using the above-described procedure except that no plant sample is rubbed between extraction pads 12 and 14. Such a negative test may be desirable to determine if the test procedure is being properly performed, and to indicate the extent, if any, to which carrier body 22 will turn a particular color by means other than a reaction with the enzyme substrate and the tag antibody-suspected antigen-capture antibody complex. A positive control test, using a liquid solution 16 known to contain the suspected antigen at an appropriate concentration, can be conducted to be sure the antigen can be detected on carrier body 22.

Various types of extraction pads may be used in the test procedure. For example, first extraction pad 12 may be a flat sheet of material secured to a backing 44 that provides support for the first extraction pad as it is rubbed against the plant sample. With this arrangement, the sap collected on pad 12 is transferred therefrom to liquid solution 16 by removing the first extraction pad from backing 44 and placing this extraction pad in the liquid solution. Preferably, tube 20 is then covered and thoroughly shaken to insure an adequate transfer of any of the suspected antigens on extraction pad 12 to liquid solution 16; and, after this, tube 20 is uncovered and extraction pad 12 is simply removed from the tube and discarded.

Preferably, both first and second extraction pads 12 and 14 are flat sheets of abrasive paper. It is desirable, though, to avoid making extraction pads 12 and 14 from materials that might break apart, or that have an abrasive that might come off, either as the plant sample is rubbed between extraction pads 12 and 14, or when pad 12 is in solution 16. With an embodiment of the diagnostic test that has been actually reduced to practice, the first extraction pad is a flat sheet of emery paper, approximately 3/4 inches by 2 1/8 inches, peelably secured to a firm cardboard backing 44, and second extraction pad 14 is a flat sheet of emery paper about 1 5/8 inches by 1 inch. In use, the plant sample 10 is simply rubbed between these first and second extraction pads 12 and 14 until the first extraction pad is thoroughly covered with sap, and then the first extraction pad is peeled from support backing 44 and placed in liquid solution 16.

Extraction pads 12 and 14 disclosed herein effectively accomplish a number of seemingly conflicting objectives. These pads 12 and 14 disrupt the physical integrity of plant sample 10 to the extent necessary to extract sap and antigen therefrom. Also, the sap and antigen are effectively collected on first extraction pad 12, eliminating the need for an additional, separate piece of equipment to hold or carry the extracted sap. At the same time, despite the abrasive nature of first extraction pad 12, antigen will pass from this pad and into solution 16, which is a form very well suited for many conventional analytical procedures. The size of first extraction pad 12 may be used to control the amount of sap added to liquid solution 16, and the size of pad 12 can be chosen to help insure an adequate amount of sap is taken so that the test results are reliable. Further, extraction pads 12 and 14 are very small, light and inexpensive.

The test method employing the dipstick of this invention is relatively simple and may be carried out by an individual without elaborate training or instructions. Also, the method is very easy to perform in the field, and does not require any expensive, heavy or cumbersome equipment. Indeed, all the equipment needed to carry out the method may be provided in a small, lightweight kit, described below, that is very easy to carry around from place to place.

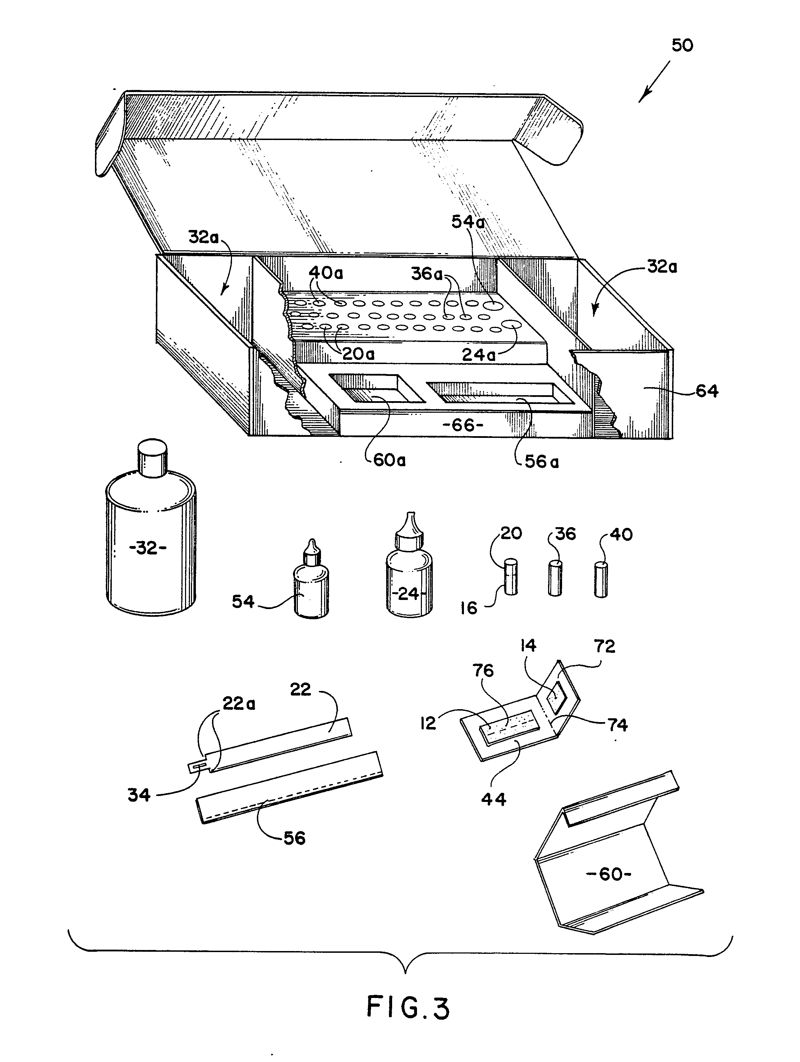

Figure 3 illustrates kit 50, including the dipstick (carrier body) of this invention, that may be employed to carry out the subject diagnostic test. Generally, the kit includes extraction pads 12 and 14, container 20 holding solution 16, and means to test that solution for the presence of the suspected antigen. This testing means includes carrier body 22 and bottle 24 holding the tag antibody, and kit 50 further includes a pair of rinse bottles 32 (only one is shown in Figure 3), rinse tube 36 and color reaction tube 40.

Extraction pads 12 and 14, solution 16, the tag antibody in bottle 24, carrier body 22, rinse bottles 32, and rinse tube 36 are used as described above so that if a suspected antigen is present in a tested plant sample, a tag antibody-antigen-capture antibody complex forms on the carrier body. The tag antibody provided in kit 50 includes a horseradish peroxidase conjugate; and, to detect that tag antibody on carrier body 22, tube 40 of kit 50 holds a peroxidase substrate, and the kit includes bottle 54 holding a source of peroxide such as a hydrogen peroxide solution.

To detect the tag antibody on carrier body 22, the solution in bottle 54 is added to the solution in tube 40 to a suitable predetermined level, and the membrane 30 on carrier body 22 is inserted into the mixed solution in tube 40. If a color is produced on membrane 30, then the tag antibody is present thereon; but if no color is produced on the membrane, then the membrane is free of the tag antibody and the suspected antigen.

A preferred embodiment of kit 50 is designed to hold supplies for ten diagnostic tests. Thus kit 50 includes ten each of pads 12 and 14, tubes 20, 36, and 40, and carrier body 22; and bottles 24, 32, 52 and 54 hold a sufficient quantity of materials for ten tests. Only one each of pads 12 and 14, tubes 20, 36 and 40, and carrier body 22 are shown in Figure 3, though, in order to simplify the figure. Each carrier body 22 may be enclosed within a first protective envelope 56, and each pair of extraction pads 12 and 14 may be enclosed within a second protective envelope 60. A plurality of bags (not shown) or other containers may be provided in kit 50 to collect and hold the plant samples. Bottles 24 and 52 may be flexible, squeeze bottles including top discharge openings; and solution from bottle 24 may simply be squeezed therefrom into solution 16, and solution from bottle 52 may be squeezed therefrom into tube 40.

Box 64 is provided to hold the components of kit 50; and styrofoam base 66 is provided to facilitate packing, shipping and handling the various pieces of equipment in the kit. Base 66 includes recesses 20a, 36a, 40a, 24a and 54a; and in an assembled position, base 66 is located in the bottom of box 64, with tubes 20, 36 and 40, respectively, located in recesses 20a, 36a and 40a, and with bottles 24 and 54, respectively, held in recesses 24a and 54a. Also, base 66 includes recess 56a for holding envelopes 56, and recess 60a for holding envelopes 60, and forms compartments 32a, at the ends of the box, for securely holding bottles 32. In addition, as will be appreciated, preferably box 64 folds to a closed position with all of the pieces of kit 50 neatly and securely held therewithin.

With the embodiment of the extraction means shown in Figure 2, first extraction pad 12 is secured to support backing 44, and second extraction pad 14 is connected to its own support backing 72. Backing 72 is connected to backing 44 to keep first and second extraction pads 12 and 14 together until they are used, and a tear line 74 may be formed at the connection between backings 44 and 72 to help tear apart those backings to use the extraction pads.

Extraction pad 12 may be provided with a central longitudinal fold line 76 so that the pad may be folded about this line to reduce its overall size to help insert the extraction pad into tube 20. In the embodiment of the invention where first extraction pad 12 is peelably removed from backing 44, preferably, when this is done, some adhesive material remains on the back surface of pad 12 --that is, the surface thereof which is in direct contact with the support backing 26. This adhesive is helpful in that it may be used to hold the two sides of the back surface together when extraction pad 12 is folded about line 76.

Liquid solution 16 is a buffer solution such as a tris-buffered saline solution; and, advantageously, the solution 16 is one which helps to extract the suspected antigen from extraction pad 12. Solution 16 may also be provided with an anti-bonding agent, commonly referred to as a blocking agent, that inhibits the attachment of the tag antibody directly to carrier body 22. Preferably, the pH of solution 16 is between about 5 and 9, and it is believed that it is best to keep the pH of this liquid solution between about 6.5 and 7.5.

The solution in container 24 is a buffered saline solution, and preferably is supplemented with proteins to protect the antibody in the solution. Preferably, the pH of the solution in container 24 is between about 5 and 9, and even more preferably, is kept between about 6.5 and 7.5. The tag antibody may be transferred to solution 16 from container 24 in any acceptable manner. For example, a small amount of solution may be aspirated into a pipette or dropper from container 24, and then dispensed into solution 16 from the pipette or dropper. After the tag antibody is added to solution 16, tube 20 is preferably capped and well shaken to insure an adequate distribution of the tag antibody throughout solution 16. The solutions in bottle 32 and tube 36 are also buffered saline solutions, and the same solution may be used in both bottle 32 and tube 36. Preferably, the pH of these solutions is between 5 and 9, and it is believed that best results are obtained if that pH is between 6.5 and 7.5.

Various alternate types of carrier bodies may be used in the practice of the diagnostic test. Dipstick type carrier bodies are preferred, though, because such sticks are very easy to handle and to wash, and have proven to be very efficient and cost effective.

Preferably, membrane 30 comprises a nitrocellulose or nylon material embedded with large quantities of antibodies, and the membrane is secured to stick 26 by a conventional adhesive such as a flat piece of tape having adhesive on both faces thereof. Care should be taken, though, to avoid using an adhesive that destroys or renders inactive the suspected antigen or the tag or capture antibodies. Any suitable technique may be employed to imbed the antibodies in membrane 30. Preferably, when or after the capture antibody is imbedded in membrane 30, the membrane is treated with a blocking agent to fill antibody attachment sites on the membrane that are not filled with the capture antibody. This blocking agent helps to keep free tag antibody in solution 16 --that is, tag antibody that has not reacted with the suspected antigen--off of the membrane 30.

A conventional reflectometer (not shown) may be provided in kit 50 to measure the intensity of the color, if any, that develops on membrane 30; and if such a meter is used, the size of the membrane should be such that it can be properly used with the meter. Lateral shoulders 22a may be formed on carrier body 22 to limit the extent to which membrane 30 can be inserted into the reflectometer, and thus to help properly position the membrane in the meter.

Various combinations of monoclonal and polyclonal antibodies may be used in the practice of the present test procedure. For instance, the tag and capture antibodies may both be monoclonal antibodies, or they may both be polyclonal antibodies. Alternately, the tag antibody may be a monoclonal antibody while the capture antibody is a polyclonal antibody, or the capture antibody may be a monoclonal antibody while the tag antibody is a polyclonal antibody.

The antibodies utilized in the practice of this diagnostic test may be made or obtained in any suitable manner. For example, U.S. Patent applications 773,811, filed April 19, 1984, and 834,198, filed August 8, 1985, disclose several procedures for making suitable monoclonal antibodies that may be used in this invention.

Preferably, the labeling enzyme used with the tag antibody is selected from the group comprising a peroxidate beta galactosidase, alkaline phosphatase or urease. Suitable enzyme substrates for a peroxidase include O-phenyl-damine, O-toluadine, 4-chloronaphthol or ABTS. Suitable enzyme substrates for beta galactosidase include para or ortho nitrophenol-d-galactosidase. Urea is a suitable substrate for urease, and paranitrophenol phosphate is a suitable substrate for alkaline phosphatase.

Numerous suitable arrangements other than the ones specifically described above, may be employed to detect the presence of the suspected antigen in solution 16. For instance, if desired, carrier body 22 may be contacted with solution 16 to develop an antigen - capture antibody complex on the carrier body, and then contacted with the tag antibody --either in solution 16 or in a separate solution --to produce the tag antibody-antigen-capture antibody complex on the carrier body. Also, if the suspected antigen is itself directly detectable on the carrier body 22, the use of the tag antibody may be eliminated.

The plant sample 10 used in the practice of the diagnostic test may be taken in any suitable way. For instance, if the plant is a grass, blades, roots or stems of the grass may be cut or pulled from the ground and used as the plant sample. Alternatively, if the plant is a tree, leaves may be pulled or cut from the tree and used as the plant sample. Regardless of the specific type of sample that is used, it should be carefully identified, and the location of the plant from which the sample is taken should be carefully recorded.

The following example illustrates the practive of the invention.

Dipsticks containing a porous membrane having an antibody immobilized therein were prepared as follows:

A. Preparation of Membranes

NOTE: Wear gloves for entire procedure.

1) Pall Biodyne Membrane is cut to the size of the vacuum filtration apparatus. The membrane is wetted with deionized water by slowly immersing one edge of the membrane and continuing until the entire membrane is wet. Try to exclude any air pockets that may become trapped in the membrane. The membrane is then soaked in Tris-buffered saline (TBS) for 15 minutes (± 5 minutes).

2) Assembly clean Schliecher and Schuell Minifold (vacuum filtration) apparatus according to alignment pegs. Place Pre-cut Blotting Paper on template, trim any excess, then place Pall membrane on top and clamp apparatus. Use just enough pressure to get the best dots and no warpage.

3) Attach vacuum and apply 400 mm Hg Vacuum while adding 400 ul TBS per well to all wells. As soon as the TBS has filtered through (about 2 minutes) turn off vacuum and disconnect hose.

4) The first or capture antibody is prepared by diluting partially purified rabbit immunoglobulin in TBS. Add 400 ul of the diluted serum to each of the 96 wells in the template. Clear all wells of air bubbles by drawing the solution into a pipettor and quickly ejecting the fluid into the wells.

5) Allow the solution to filter through (by gravity) until the wells are empty.

6) Turn on vacuum (connect it) and allow it to vacuum dry (400 mm Hg vacuum) for 15 minutes. With vacuum on, dissemble apparatus and remove membrane with forceps.

7) Hang membrane by one corner and allow to hang dry at room temperature for ca. 1 hour.

8) Re-wet membrane in deionized water and then transfer to Blocking solution which has been heated to 40°C. Allow this to incubate in Blocker for 30 minutes with some agitation of the solution. Change Blocking Solution after 30 minutes and repeat this procedure. (4 changes of Blocker, 2 hours total time).

9) After blocking, remove membrane with forceps, hang by one corner and allow it to dry overnight (ca. 16 hrs.) at room temperature.

10) Store membranes in container with dessicant separated by layers of adsorbent paper until it is attached to dipsticks.

B. Preparation of Dipsticks

1) Wash dipstick blanks with deionized water and dry on paper towels.

2) Attach double stick tape (3M brand carpet tape) to narrow end of dipstick and trim excess with scissors. Leave protective green coating in place.

3) Handle the prepared membrane only with gloves and forceps. On a clean, dry surface, cut the membrane with scissors into individual squares so that each contains one "dot" of antibody.

4) With sharp forceps, remove green protective coating from tape on dipstick and then place a single dot on the tape, centered and 6 mm from the bottom of the dipstick (use of forceps and template which marks 6 mm point make this more accurate).

5) Using flat forceps, press dot firmly onto the tape.

6) Trim excess with scissors.

7) Dipsticks are then stored in open test tubes on a rack at room temperature.

## C. Specification and Recipes

1) Membranes

Pall Biodyne Affinity Membrane
0.65 um pore size
Pall Corporation #BIA0065C5

2) Tris Buffered Saline (TBS) 500 mM NaCl; 50 mM Tris; pH 7.5

|  | Per Liter |
|---|---|
| NaCl | 29.22 g |
| Trizma Base | 1.18 g |
| Trizma HCl | 6.35 g |

pH to 7.5 using Trizma Base or Trizma HCl

3) Vacuum Filtration Device

Schleicher and Schuell Minifold
#5RC-096/0

4) Blotting Paper

Schleicher and Schuell Pre-Cut Blotting Paper (Grade GB002) cut 14 x 12 cm
#32-17470

5) Capture (First Antibody) - Ammonium Sulfate Precipitated Polyclonal Rabbit Serum

Dilute 1:1000* in Tris Buffered Saline
*adjusted according to antibody lot

6) Blocking Solution

5% Blotto diluted in TBS

|  | Per Liter |
|---|---|
| Carnation Non-Fat dry milk | 50 g |
| Antifoam A | 0.1 ml |

Heat to 40°C before using

While it is apparent that the invention disclosed herein is well calculated to fulfill the objects previously stated, it will be appreciated that numerous modifications and embodiments may be devised by those skilled in the art, and it is intended that the appended claims cover all such modifications and embodiments as fall within the true spirit and scope of the present invention.

## Claims

1. A process of preparing a dipstick (2) having antibodies immobilized thereon, characterized by:

(a) contacting a porous membrane (6), effective to covalently bond with antibodies, with a solution of antibodies under conditions effective to covalently bond said antibodies to said porous membrane (6),

(b) drying said porous membrane (6), and

(c) affixing said porous membrane (6) on a longitudinal end of an elongated substrate (4).

2. A process according to claim 1, characterized by the following steps, conducted after step (b) and before step (c) contacting said porous membrane (6), having antibodies bonded thereto, with a blocking solution under conditions effective to inactivate sites on said membrane (6) otherwise available for bonding with antibodies, and drying said porous membrane (6).

3. A process according to claim 2, characterized in that the blocking solution. is a buffered saline solution of non-fat milk.

4. A process according to claims 1-3, characterized in that step (c) is performed with an adhesive means (8).

5. A process according to claim 4 characterized in that the adhesive means (8) comprises a tape including a first face having an adhesive and connected to the elongated substrate (4) and a second face, opposite the first face, having an adhesive and connected to the porous membrane (6).

6. A process according to claims 1-5, characterized in that the porous membrane (6) is an activated nylon membrane.

7. A process according to claims 1-6, characterized by the following steps conducted prior to step (a) contacting the porous membrane (6) with deionized water, contacting the wet, porous membrane (6) with saline solution buffered to pH of about 7.5, and removing excess liquid from the porous membrane (6).

8. A process according to claims 1-7, characterized in that the elongated substrate (4) is composed of a non-wetting plastic material.

9. A process according to claim 8, characterized in that the plastic material is polypropylene, poly (ethylene terphthalate) or poly (vinyl chloride).

10. A process according to claims 1-9, characterized in that step (c), the porous membrane (6) is affixed so that said membrane (6) is centrally located midway between the longitudinally extended sides of said elongated substrate (4).

11. A dipstick having antibodies bound thereto prepared by the process of any one of claims 1 to 10.

# FIG. IA

IB 2 4 6 IB

# FIG. IB

2 4 6

B

FIG.2

0 246 643

FIG.3